# EUROPEAN PATENT SPECIFICATION

(11) **EP 3 060 156 B1**
(45) Date of publication and mention of the grant of the patent: **03.04.2019**
(21) Application number: 14784428.6
(22) Date of filing: 13.10.2014
(51) Int. Cl.: A61B 90/00, A61B 8/08, A61B 5/01, A61B 18/14

(54) **TEMPERATURE MONITORING APPARATUS FOR MONITORING A TEMPERATURE WITHIN A TISSUE**
TEMPERATURÜBERWACHUNGSVORRICHTUNG ZUR ÜBERWACHUNG EINER TEMPERATUR IN EINEM GEWEBE
APPAREIL DE CONTRÔLE DE TEMPÉRATURE POUR SURVEILLER UNE TEMPÉRATURE À L'INTÉRIEUR D'UN TISSU

(30) Priority: 22.10.2013 US 201361893921 P; 19.11.2013 EP 13193522
(43) Date of publication of application: 31.08.2016
(73) Proprietor: Koninklijke Philips N.V., 5656 AE Eindhoven (NL)
(72) Inventor: ANAND, Ajay, 5656 AE Eindhoven (NL); SETHURAMAN, Shriram, 5656 AE Eindhoven (NL); ZHOU, Shiwei, 5656 AE Eindhoven (NL); XIE, Hua, 5656 AE Eindhoven (NL); LI, Junbo, 5656 AE Eindhoven (NL); ROBERT, Jean-luc, 5656 AE Eindhoven (NL)
(74) Representative: Gravendeel, Cornelis
(86) International application number: PCT/EP2014/071825
(87) International publication number: WO 2015/058981

(56) References cited:
- EP-A1- 2 387 963
- US-B2- 7 520 877
- B ARNAL ET AL: "Monitoring of thermal therapy based on shear modulus changes: I. shear wave thermometry", IEEE TRANSACTIONS ON ULTRASONICS, FERROELECTRICS AND FREQUENCY CONTROL, vol. 58, no. 2, 1 February 2011 (2011-02-01), pages 369-378, XP055111822, ISSN: 0885-3010, DOI: 10.1109/TUFFC.2011.1814 cited in the application
- ANAND A ET AL: "Three-dimensional spatial and temporal temperature imaging in gel phantoms using backscattered ultrasound", IEEE TRANSACTIONS ON ULTRASONICS, FERROELECTRICS AND FREQUENCY CONTROL, IEEE, US, vol. 54, no. 1, 1 January 2007 (2007-01-01), pages 23-31, XP011381542, ISSN: 0885-3010, DOI: 10.1109/TUFFC.2007.208 cited in the application
- ARNAL B ET AL: "Monitoring of thermal therapy based on shear modulus changes: II. Shear wave imaging of thermal lesions", IEEE TRANSACTIONS ON ULTRASONICS, FERROELECTRICS AND FREQUENCY CONTROL, IEEE, US, vol. 58, no. 8, 1 August 2011 (2011-08-01) , pages 1603-1611, XP011386889, ISSN: 0885-3010, DOI: 10.1109/TUFFC.2011.1987

## Description

### FIELD OF THE INVENTION

The present invention relates to a temperature monitoring apparatus for monitoring a temperature within a tissue during a thermal ablation process and to a temperature monitoring method for monitoring a temperature within a tissue during a thermal ablation process.

### BACKGROUND OF THE INVENTION

In the field of minimally invasive surgeries thermal ablation techniques are well known and provide excellent alternatives to major surgeries. This technique merely requires needles or probes for providing a radiofrequency, cryotherapy and/or microwave ablation. Alternatively a non-invasive heat source can be used such as using ultrasound. In each case, tissue is coagulated by heating the tissue to a temperature above 60 °C. This technique is preferably used to treat cancer.

Radiofrequency ablation is currently the most common minimally invasive heating therapy to treat cancer, in particular liver cancer. The radiofrequency ablation uses a probe with an active electrode tip through which a high frequency alternating current is conducted. The current propagates through the body to a grounding pad, wherein the current causes ionic agitation and frictional heating. The heat introduced into the tissue is dissipated through thermal conduction to ablate the tumor.

Due to a high recurrence rate of cancer, follow up inspections are done within a month and again at three months intervals along with tumor markers to detect residual disease or recurrence. One common reason for the high recurrence rate is the inability to monitor and to control the ablation size of the thermal ablation technique to adequately destroy all tumor cells. It is therefore essential to provide a real-time feedback by means of a temperature map of the ablated zone.

A temperature profile of the surgery can currently be achieved by magnet resonance-based temperature imaging, however, this technique is expensive and may not be readily available.

Alternatively ultrasound thermometry can be used for thermal ablation monitoring, however, since the sensitivity of this method is significantly reduced above 50 °C and since the ultrasound echoes significantly change with the onset of tissue necrosis, a precise temperature measurement and the generations of a precise temperature map of the ablation zone is not possible.

Alternatively ultrasound shear wave imaging is a well known technique to inspect cancer and to provide a contrast between ablated tissue and the surrounding normal tissue to monitor the thermal ablation in real time. However, the heat introduction during radio frequency microwave ablation forms gas bubbles around the core of the ablation zone, which can introduce artifacts in the shear wave imaging so that the thermal monitoring of the ablation can be disturbed.

Both ultrasound inspection methods using ultrasound thermometry or shear wave imaging can be disturbed by metal devices so that the monitoring close to the core of the ablation zone is not possible due to the presence of the radio frequency ablation probe.

Alternative methods dispose the shear wave imaging planes on the periphery of the ablation zone and determine the temperature of the ablation zone by using a thermal model, however, due to the inhomogeneity of the tissue due to blood vessels, which act as heat sinks, a precise determination of the temperature in the ablation zone is not possible.

US 2010/0256530 A1 discloses a method and an apparatus for monitoring tissue ablation, wherein the position of the ablation electrodes is monitored by an ultrasound transducer, however, due to the metal electrodes and the temperature and gas bubble effects in the ablation zone, a precise temperature monitoring of the ablation zone is not possible.

EP 2 387 963 A1 discloses a temperature distribution determining apparatus for determining a temperature distribution within an object caused by applying energy to the object, wherein an ultrasound unit measures a spatially and temporally dependent first temperature distribution of the object while the energy is applied to the object such that the object is heated to a temperature within a first temperature range, and a temperature distribution estimating unit estimates a spatially and temporally dependent second temperature distribution in the object within a second temperature range, which is different to the first temperature range.

From B. Arnal et al.: "Monitoring of Thermal Therapy Based on Shear Modulus Changes: I. Shear Wave Thermometry", in IEEE transactions on ultrasonics, ferroelectrics and frequency control, Vol. 58, No. 2, 1 February 2011, pages 369-378, a method for determining a temperature of a tissue on the basis of shear wave thermometry is known.

From A. Anand et al.: "Three-Dimensional Spatial and Temporal temperature Imaging in Gel Phantoms Using Backscattered Ultrasound", in IEEE transactions on ultrasonics, ferroelectrics and frequency control, Vol. 54, No 1, 1 January 2007, pages 23-31 a method for determining a temperature in a tissue on the basis of backscattered ultrasound is known.

Further prior art on shear wave thermometry is found in B. Arnal et al.: "Monitoring of thermal therapy based on shear modulus changes: II. Shear wave imaging of thermal lesions", in IEEE transactions on ultrasonics, ferroelectrics and frequency control, Vol. 58, No 8, 1 August 2011, pages 1603-1611.

US 7 520 877 B2 discloses a radio frequency system using multiple prong probes, wherein the prongs are electrically isolated from each other and wherein electrical power is rapidly switched between the prongs, between a prong and a ground pad or both.

### SUMMARY OF THE INVENTION

It is therefore an object of the present invention to provide an improved temperature monitoring apparatus for monitoring a temperature within a tissue having a high precision with low technical effort.

According to the present invention a temperature monitoring apparatus is provided for monitoring a temperature within a tissue during a thermal ablation process, comprising:
- a temperature application unit configured to introduce heating power into the tissue for heating the tissue,
- an ultrasound unit configured to emit and receive ultrasound waves and configured to determine a temperature in a measurement region of the tissue on the basis of ultrasound shear wave detection during the ablation process, and
- a temperature estimation unit including a heat transfer model configured to estimate a temperature in a region of interest within the tissue on the basis of the determined temperature and the heat transfer model, wherein the heat transfer model is based on medical images of the tissue.

Provided is furthermore a method, not according to the invention, for monitoring a temperature within a tissue during an ablation process, comprising the steps of:
- introducing a heating power into the tissue for heating the tissue,
- determining a temperature in a measurement region of the tissue on the basis of ultrasound shear wave detection during the ablation process, and
- estimating a temperature in a region of interest within the tissue on the basis of a heat transfer model and the determined temperature, wherein the heat transfer model is based on medical images of the tissue.

Preferred embodiments of the invention are defined in the dependent claims. It shall be understood that the claimed method has similar and/or identical preferred embodiments as the claimed device and as defined in the dependent claims.

The present invention is based on the idea to measure the temperature of the tissue non-invasive separately from the position where the heating power is introduced into the tissue and to estimate the temperature of the tissue in a region of interest, e.g. in a region between the position of power introduction and the measurement region so that a temperature map can be provided and an ablation zone can be precisely determined. A heat transfer model is used by the temperature estimation unit in order to estimate the temperature in the region of interest and to improve the estimation, parameters of the transfer model are determined on the basis of medical images such as computer tomography images, ultrasound images or magnet resonance images. Since the medical images are determined by the properties of the tissue, the heat transfer model can be precisely determined so that the temperature within the region of interest can be precisely estimated without additional probing or measurement units. Hence, the monitoring of the temperature within the region of interest is possible with high precision and low technical effort.

In a preferred embodiment, the ultrasound unit is adapted to define the measurement region within the tissue on the basis of the medical images. This is a possibility to define an optimal position within the tissue in order to provide a precise estimation of the temperature within the region of interest.

In a preferred embodiment, the temperature application unit is adapted to determine the heating power introduced into the tissue on the basis of the medical images. This is a possibility to precisely predetermine the temperature of the tissue heated by the heating power, since the thermal properties of the tissue can be derived from the medical images.

In a preferred embodiment, the measurement region comprises at least one measurement plane. This is a possibility to precisely define a position of the measurement region since the distance of the measurement plane can be precisely located in a defined distance from the region of interest.

In a preferred embodiment, the measurement region is formed separately from the region of interest. This is a possibility to increase the sensitivity of the ultrasound shear wave temperature detection, since the influence of the high temperature effects on the ultrasound measurement in the region of interest can be reduced.

In a preferred embodiment, the temperature estimation unit comprises a position sensor for determining a position of the region of interest and a position of the measurement region. This is a possibility to precisely determine the distance of the region of interest and the measurement region so that a precise estimation of the temperature within the region of interest can be achieved.

In a preferred embodiment, the temperature estimation unit is adapted to detect heat sinks within the tissue on the basis of the medical images and wherein the temperature estimation unit is configured to adapt the heat transfer model on the basis of the detected heat sinks. This is a possibility to improve the performance of the heat transfer model, since blood vessels which can form heat sinks within the tissue can be easily detected by means of the medical images.

In a preferred embodiment, the temperature estimation unit is configured to adapt parameters of the heat transfer model on the basis of ultrasound temperature measurements in different measurement regions. This is a possibility to compare estimated temperatures with ultrasound temperature measurement and to improve the heat transfer model continuously by adapting the respective model parameters.

In a certain embodiment, the ultrasound temperature measurements are performed in the periphery of the ablation zone.

The heat transfer model parameter are determined based on the shear wave measurements performed during or prior to the ablation. Hence, an adaption of the heat transfer model in real-time is possible.

In a preferred embodiment, the temperature application unit is adapted to control the heating power introduced into the tissue on the basis of the estimated temperature in the region of interest. This is a possibility to set the temperature within the region of interest to a predefined level, so that a precise treatment of the tissue is possible.

In a preferred embodiment, the ultrasound unit is adapted to define a position of the measurement region within the tissue on the basis of an expected temperature of the tissue determined on the basis of the heat transfer model. This is a possibility to define the measurement region in a position having a tissue temperature, which is within a high sensitivity range of the shear wave detection, so that the sensitivity of the temperature measurement can be improved.

In a preferred embodiment, the temperature application unit is adapted to introduce a heating power pulse into the tissue, wherein the temperature estimation unit is adapted to determine parameters of the heat transfer model on the basis of a temperature determined by the ultrasound unit in the measurement region generated by the heating power pulse. This is a possibility to determine parameters of the heat transfer model prior to the treatment on the basis of a real measurement within the tissue to be monitored so that a precise treatment of the tissue can be achieved.

In a preferred embodiment, the temperature application unit comprises a probe for introducing the heating power into the tissue, wherein the probe comprises a temperature measurement device for measuring the temperature of the tissue. This is a possibility to further improve the temperature measurement and the heat transfer model, since a further real temperature measurement can be provided.

It is further preferred if the temperature application unit is adapted to introduce a heating power pulse into the tissue, wherein the ultrasound unit is adapted to calibrate the ultrasound temperature measurement on the basis of the temperature measured by the temperature measurement device of the probe. This is a possibility to improve the ultrasound measurement, since the ultrasound unit can be calibrated on the basis of an in-situ temperature measurement. In this case, it is preferred if the measurement region is formed in close vicinity of the probe, so that thermal conductivity effect can be minimized.

In a preferred embodiment, the medical images are ultrasound images, computer tomography images and/or magnet resonance tomography images. This is a possibility to provide precise medical images in order to improve the temperature estimation on the basis of the heated transfer model.

As mentioned above, the present invention is based on the idea to estimate the temperature within the region of interest on the basis of a heat transfer model, wherein the heat transfer model is adapted by means of medical images of the respective tissue. Therefore, the heat transfer model can be adapted to the real tissue properties, in particular to the presence of the blood vessels, which act as heat sinks within the tissue and which can disturb the estimation of the temperature within the region of interest. Hence, a precise estimation of the temperature within the region of interest is possible with low technical effort, so that a precise ablation treatment of tissue is possible.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of an ablation zone and an ultrasound temperature measurement;
Fig. 2 shows a schematic block diagram of a temperature monitoring apparatus; and
Fig. 3 shows a schematic block diagram of a temperature monitoring and treatment method.

### DETAILED DESCRIPTION OF THE INVENTION

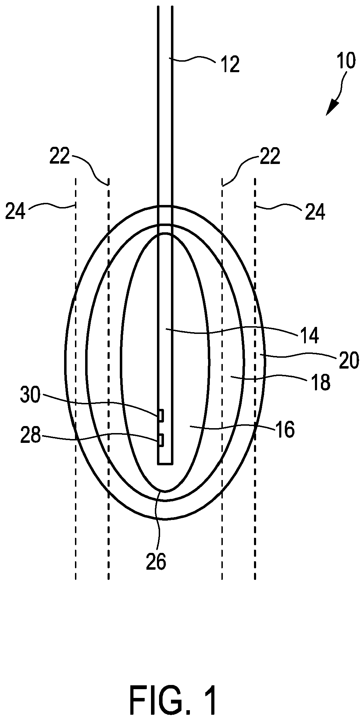

Fig. 1 shows a tissue e.g. of a human being generally denoted by 10. In the tissue 10, a temperature application device 12 having a probe or a tip 14 is disposed for heating the tissue 10 to a predefined temperature in order to coagulate parts of the tissue 10 or to ablate parts of the tissue 10. The temperature application device 12 is preferably a radio frequency application unit, a cryotherapy unit or a microwave ablation unit as minimally invasive techniques or may alternatively be a non-invasive heat source such as using ultrasound (HIFU). The temperature application device 12 introduces heating power into the tissue 10 so that the tissue 10 is heated to a temperature above 60 °C and that the tissue 10 in an ablation zone 16 is ablated or coagulated. Due to the thermal conductivity of the tissue 10, the ablation zone 16 is surrounded by medium temperature zone 18, wherein the tissue 10 in the medium temperature zone 18 is heated to a medium temperature lower than the temperature in the ablation zone 16. The medium temperature zone 18 is surrounded by a low temperature zone 20, wherein the tissue 10 of the low temperature zone 20 is lower than the temperature of the medium temperature zone 18. The medium temperature zone 18 is heated by means of the heating power of the temperature application device 12 to a temperature of 50-60 °C so that no coagulation or ablation occurs in this medium temperature zone 18. The low temperature zone 20 is heated by means of the heating power of the temperature application device 12 to a temperature below 50 °C.

The temperature of the tissue 10 is measured by means of ultrasound shear wave detection in different measurement positions 22, 24 within the tissue 10 and preferably separated from the ablation zone 16. The measurement positions 22, 24 are usually different measurement planes 22, 24 which are disposed within the tissue at different distances from the ablation zone 16. The ultrasound shear wave detection is preferably performed by ultrasound shear wave thermometry (SWT) or ultrasound shear wave imaging (SWI). Since the shear wave thermometry is applicable for tissue temperatures up to 60 °C, the temperature measurement positions 22, 24 should be placed separately from the ablation zone 16 in order to provide precise measurement results. The ultrasound shear wave imaging is applicable up to 65 °C so that the measurement positions 22, 24 may be disposed closer to the ablation zone, however, due to the measurement disturbances like gas bubbles as mentioned above, the measurement position 22, 24 should be separated from the ablation zone 16 in one of the temperature zones 18, 20 having a tissue temperature below 60 °C.

To provide a precise treatment of the tissue, in particular a precise treatment of cancer like liver cancer, the boundary of the ablation zone 16 or the three-dimensional size of the ablation zone 16 has to be determined precisely within the tissue 10 to remove e.g. all tumor cells and to reduce the risk of cancer recurrence. Hence, the boundary of the ablation zone 16 is determined as a region of interest 26 on the basis of the ultrasound shear wave measurements at the measurement positions 22, 24 and a bio-heat transfer model by means of which a three-dimensional temperature map is determined. The bio-heat transfer model is based on medical images of the tissue 10 derived from independent imaging modalities like computer tomography, ultrasound or magnet resonance technology, wherein the medical images are taken or performed before the ablation procedure. On the basis of the medical images, the present properties of the tissue 10 like location of blood vessels, which form heat sinks within the tissue 10 are determined and imported to the bio-heat transfer model so that a precise estimation of the temperature distribution within the tissue 10 is possible.

The map of the position of the blood vessels is provided initially prior to the ablation process. Since the thermal and electrical properties of the tissue 10 are unknown properties at the beginning of the ablation, these parameters are estimated during the ablation process as described in the following. The measurement data of the ultrasound shear wave detection is continuously provided to the heat transfer model in order to adapt the model and to continuously adapt the specific parameters of the model. Therefore, a precise temperature distribution in all zones 16, 18, 20 of the tissue 10 can be determined.

In a preferred embodiment, the estimated temperature map is evaluated and the heating power provided by the temperature application device 12 into the tissue 10 is controlled or modified in order to optimize the thermal treatment and to control the size of the ablation zone 16.

Hence, by means of the ultrasound shear wave measurements in the measurement planes 22, 24, the temperature within the region of interest 26 can be precisely estimated so that a precise heat treatment of the tissue 10 is possible.

In a pretreatment phase, ultrasound guidance is generally used to identify the position of the probe 14 and can be used to define the position of the measurement planes 22, 24 for ultrasound shear wave detection. For the position detection of the probe 14, the probe 14 may comprise a position sensor 28, which may be a tracking sensor on the basis of electromagnetic, ultrasonic or optical waves. The distance between the probe 14 and the measurement plane 22, 24 may also be set or controlled by means of a mechanical fixture coupled to the temperature application device 12 and to the respective ultrasound shear wave unit.

The medical images are also utilized to define the measurement positions 22, 24 in order to avoid the propagation of the ultrasound shear waves through blood vessels in order to avoid artifacts during the ultrasound shear wave measurements.

When the measurement positions 22, 24 are defined and adjusted, the thermal model is initialized and the treatment is started. Usually the model utilizes initial values for the tissue parameters derived e.g. from literature values. For example, the electrical conductivity is σ = 0,148 S/m, the thermal conductivity is 0,465 W/m °C, the density is 1060 kg/m³, the heat capacity is 3600 J/C kg, the perfusion rate is 6,4 x 10⁻³/sec. The heating power is applied as electrical power and an impedance profile is acquired and imported in real time into the thermal model.

During the process, the measurement planes 22, 24 are used to generate shear wave imaging based temperature estimation. In parallel, the temperature estimated by the thermal model is compared with the temperature obtained by the shear wave thermometry as well as temperature measurements performed by a temperature sensor 30 provided at the tip 14 of the temperature application device 12. By means of these measurements and the comparison of the estimated temperature with the measured temperatures, the parameters of the thermal model are constantly and continuously adapted to minimize the difference between the model prediction and the experimental data. The adapted parameters updated during the ablation procedure include thermal constants such as thermal diffusivity and perfusion rate, electrical properties such as electrical conductivity and average flow rate in the blood vessels visualized in the computer tomography or ultrasound images. By means of this flexibility of the thermal model, e.g. for local heterogeneities, the temperature map and the ablation zone 16 can be precisely determined so that the boundary of the ablation zone 16 as the region of interest can be precisely measured non-invasively in order to optimize the thermal treatment.

The so derived temperature distribution may guide the operator of the temperature application unit in order to complete the treatment or adapt the treatment and could also be used as a feedback to control the power output of the thermal application device 12.

The thermal model or the bio-heat transfer model is preferably an implementation of the Pennes' bio-heat equation that takes the heat diffusion in the tissue 10 and the blood perfusion resulting from the applied heat source into account. Additional heat transfer equation considering heat sink effects due to directional flow in larger blood vessels can also be incorporated into the thermal model. In addition to the bio-heat transfer, the underlying physics for the heat deposition (i.e. heat source) can be modeled using the corresponding physics interface like electrical currents interface for radio frequency heating. Multi-physics simulations can be applied to model the tissue heating during the radio frequency ablation treatments.

The local in situ tissue specific parameters for the heat transfer model are determined based on the shear wave measurements performed during or immediately before the ablative therapy to adapt the heat transfer model in real-time.

The heat transfer model parameters may be determined or estimated in advance of the ablation process. In a first step, a heating power pulse is applied by the probe 14 to the tissue 10 and the induced temperature distribution is measured at the measurement planes 22, 24 by means of the ultrasound shear wave measurements. The measured temperatures and the heating power pulse are used to estimate or determine the unknown model parameters. After the parameters are determined, the expected size of the ablation zone 16 for the heating power profile to be used during the ablation process can be determined. Possible deviations between the estimated and the determined size of the ablation zone 16 can be adapted accordingly. This embodiment reduces the need for real time instant estimation of the thermal model parameters during the ablation process.

In a certain embodiment, the tissue temperature is used, which is estimated from the shear wave measurements performed in the periphery of the ablation zone 16.

In a further embodiment the ultrasound shear wave measurement is calibrated prior to the ablation process. During the calibration step, the measurement planes 22, 24 are disposed close to the temperature sensor 30 of the probe 14 and a heating power pulse is applied by means of the temperature application device 12. The ultrasound shear wave measurement and the temperature measurement of the temperature sensor 30 are compared and the ultrasound shear wave measurement is calibrated according to the temperature sensor measurement. By means of this calibration, also the tissue composition close to the probe 14 can be considered. The so derived parameters are fed to the thermal model in order to determine model parameters prior to the ablation process.

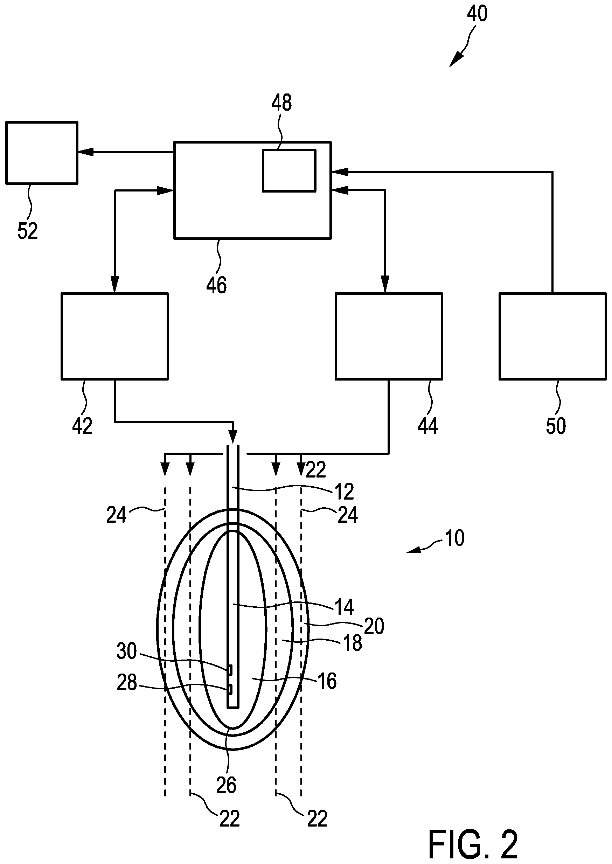

In Fig. 2, a temperature monitoring apparatus is schematically shown and generally denoted by 40. The temperature monitoring apparatus 40 comprises a temperature application unit 42 comprising the temperature application device 12 for applying the heating power to the probe 14 in order to heat the tissue 10 and to perform the ablation process of the tissue 10. The temperature monitoring apparatus 40 further comprises an ultrasound unit 44 for emitting and receiving ultrasound waves and for determining a temperature at the measurement positions 22, 24 of the tissue 10 on the basis of ultrasound shear wave detection. The ultrasound unit 44 provides shear wave thermometry (SWT) and/or shear wave imaging (SWI) in order to determine a temperature at the measurement positions 22, 24. The ultrasound unit 44 is further adapted to define the measurement positions 22, 24 within the tissue 10 with respect to the position of the probe 14. The ultrasound unit 44 further comprises a position sensor to determine the measurement positions 22, 24 with respect to the position of the probe 14.

The temperature monitoring apparatus 40 further comprises a temperature estimation unit 46 including a heat transfer model 48. The temperature estimation unit 46 is connected to an imaging unit 50 for receiving the medical images from the tissue 10 to be treated. The temperature estimation unit 46 is further connected to the temperature application unit 42 and to the ultrasound unit 44 for receiving measurement data and for controlling the heating power applied to the tissue 10. The temperature estimation unit 46 is adapted to estimate the temperature in the region of interest 26 within the tissue 10 on the basis of the heat transfer model 48 and the measurements of the ultrasound unit 44 at the measurement positions 22, 24, wherein the heat transfer model 48 is based on the medical images received from the image unit 50. Hence, local properties of the tissue 10 can be considered for estimating the temperature within the tissue 10 in general and in particular within the region of interest 26 in order to precisely determine the ablation of the tissue 10.

The temperature estimation unit 46 may be connected to an output device 52 for providing the estimated temperature and in particular the estimated temperature map of the tissue 10 to an operator. The temperature monitoring apparatus 40 may also comprise an operator control (not shown) coupled to the temperature application unit 42 that allows the operator to adjust or terminate the ablation process.

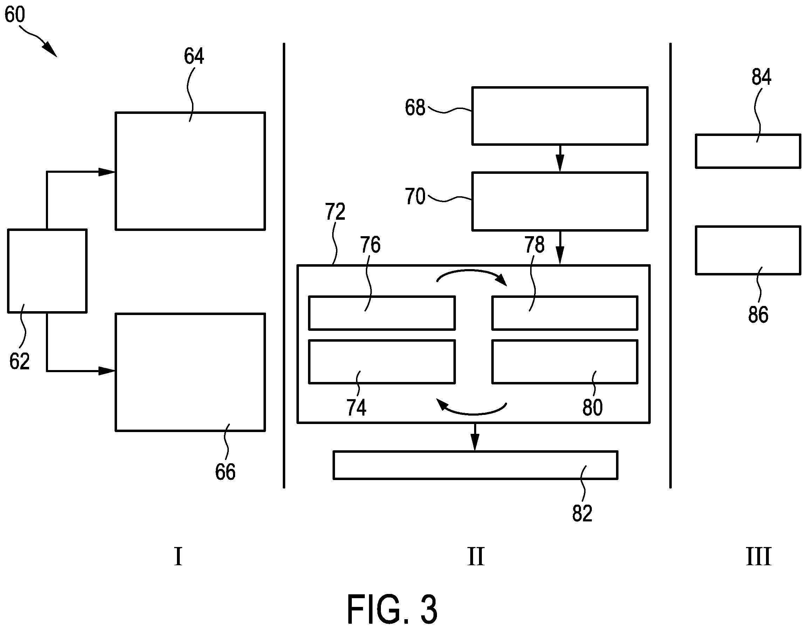

In Fig. 3 a schematic flow diagram of the temperature monitoring method is shown and generally denoted by 60.

In general, the temperature monitoring method 60 generally comprises three phases, a pretreatment phase I, a treatment phase II and a post-treatment phase III.

During the pretreatment phase I, the medical images are captured 62 and the measurement planes 22, 24 are disposed within the tissue 10 apart from the ablation zone 16 at a known distance using position tracking methods, e.g. by means of the position sensor 28 as shown at 64. At 66, the thermal model is initialized by means of the e.g. the blood vessel information received from the medical images as shown at 66. At 66, further parameter of the thermal model can be determined e.g. on the basis of the perfusion, the directional flow, the tissue properties and the properties of the probe 14 derived from the medical images.

During the treatment phase II, the measurement planes 22, 24 are placed around the region of interest 26 as shown at 68 and the temperature in these measurement planes 22, 24 is measured by means of the shear wave thermometry as shown at 70.

The thermal model is continuously updated and improved during the treatment phase II as generally shown at 72. During this adaption 72 of the thermal model 48, the thermal model 48 is utilized as shown at 74 and the temperature distribution in the tissue 10 is estimated as shown at 76. The estimated temperatures are compared with the temperature measurements at the measurement planes 22, 24 as shown at 78 and the model parameter are adapted to match the temperatures measured by means of the shear wave thermometry as shown at 80. This update 72 of the thermal model 48 is a continuous process during the hole treatment phase II. As a result, a temperature estimation over the whole volume of the tissue 10 is provided as shown at 82.

During the post-treatment phase III, the feedback of the ablation process is provided to the operator as shown at 84 and a decision whether the ablation process should be continued or terminated can be provided as shown at 86.

Consequently, the whole ablation process can be optimized by providing a precise temperature map of the tissue 10.

While the invention has been illustrated and described in detail in the drawings and foregoing description, such illustration and description are to be considered illustrative or exemplary and not restrictive; the invention is not limited to the disclosed embodiments but to the appended independent claim. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. Temperature monitoring apparatus (40) for monitoring a temperature within a tissue (10) during a thermal ablation process, comprising:
- a temperature application unit (42) configured to introduce heating power into the tissue for heating the tissue,
- an ultrasound unit (44) configured to emit and receive ultrasound waves and configured to determine a temperature in a measurement region (22, 24) of the tissue on the basis of ultrasound shear wave detection during the ablation process, and
- a temperature estimation unit (46) including a heat transfer model (48) configured to estimate a temperature in a region of interest (26) within the tissue on the basis of the determined temperature and the heat transfer model, wherein the heat transfer model is based on medical images of the tissue.

2. Temperature monitoring apparatus as claimed in claim 1, wherein the ultrasound unit is adapted to define the measurement region within the tissue on the basis of the medical images.

3. Temperature monitoring apparatus as claimed in claim 1, wherein the temperature application unit is adapted to determine the heating power introduced into the tissue on the basis of the medical images.

4. Temperature monitoring apparatus as claimed in claim 1, wherein the measurement region comprises at least one measurement plane.

5. Temperature monitoring apparatus as claimed in claim 1, wherein the measurement region is formed separately from the region of interest.

6. Temperature monitoring apparatus as claimed in claim 1, wherein the temperature estimation unit comprises a position sensor (28) for determining a position of the region of interest and a position of the measurement region.

7. Temperature monitoring apparatus as claimed in claim 1, wherein the temperature estimation unit is adapted to detect heat sinks within the tissue on the basis of the medical images and wherein the temperature estimation unit is configured to adapt the heat transfer model on the basis of the detected heat sinks.

8. Temperature monitoring apparatus as claimed in claim 1, wherein the temperature estimation unit is configured to adapt parameters of the heat transfer model on the basis of ultrasound temperature measurements in different measurement regions.

9. Temperature monitoring apparatus as claimed in claim 1, wherein the temperature application unit is adapted to control the heating power introduced into the tissue on the basis of the estimated temperature in the region of interest.

10. Temperature monitoring apparatus as claimed in claim 1, wherein the ultrasound unit is adapted to define a position of the measurement region within the tissue on the basis of an expected temperature of the tissue determined on the basis of the heat transfer model.

11. Temperature monitoring apparatus as claimed in claim 1, wherein the temperature application unit is adapted to introduce a heating power pulse into the tissue and wherein the temperature estimation unit is adapted to determine parameters of the heat transfer model on the basis of a temperature determined by the ultrasound unit in the measurement region generated by the heating power pulse.

12. Temperature monitoring apparatus as claimed in claim 1, wherein the temperature application unit comprises a probe (14) for introducing the heating power into the tissue, wherein the probe comprises a temperature measurement device (30) for measuring the temperature of the tissue.

13. Temperature monitoring apparatus as claimed in claim 12, wherein the temperature application unit is adapted to introduce a heating power pulse into the tissue and wherein the ultrasound unit is adapted to calibrate the ultrasound temperature measurement on the basis of the temperature measured by the temperature measurement device of the probe.

14. Temperature monitoring apparatus as claimed in claim 1, wherein the medical images are ultrasound images, computer tomography images and/or magnet resonance tomography images provided by an image unit (50).

## Patentansprüche

1. Temperaturüberwachungsvorrichtung (40) zum Überwachen einer Temperatur in einem Gewebe (10) während eines Thermoablationsprozesses, umfassend:
- eine Temperaturbeaufschlagungseinheit (42), die konfiguriert ist, um Heizleistung in das Gewebe einzubringen, um das Gewebe zu erwärmen,
- eine Ultraschalleinheit (44), die konfiguriert ist, um Ultraschallwellen auszusenden und zu empfangen, und die konfiguriert ist, um eine Temperatur in einem Messbereich (22, 24) des Gewebes auf der Grundlage der Ultraschallschubwellenerfassung während des Ablationsprozesses zu bestimmen, und
- eine Temperaturschätzeinheit (46) beinhaltend einem Wärmeübertragungsmodell (48), das konfiguriert ist, um eine Temperatur in einem Bereich von Interesse (26) innerhalb des Gewebes auf der Grundlage der bestimmten Temperatur und des Wärmeübertragungsmodells zu schätzen, wobei das Wärmeübertragungsmodell auf medizinischen Bildern des Gewebes basiert.

2. Temperaturüberwachungsvorrichtung nach Anspruch 1, wobei die Ultraschalleinheit angepasst ist, um den Messbereich innerhalb des Gewebes auf der Grundlage der medizinischen Bilder zu definieren.

3. Temperaturüberwachungsvorrichtung nach Anspruch 1, wobei die Temperaturbeaufschlagungseinheit angepasst ist, um die in das Gewebe eingebrachte Heizleistung auf der Grundlage der medizinischen Bilder zu bestimmen.

4. Temperaturüberwachungsvorrichtung nach Anspruch 1, wobei der Messbereich mindestens eine Messebene umfasst.

5. Temperaturüberwachungsvorrichtung nach Anspruch 1, wobei der Messbereich getrennt von dem Bereich von Interesse ausgebildet ist.

6. Temperaturüberwachungsvorrichtung nach Anspruch 1, wobei die Temperaturschätzeinheit einen Positionssensor (28) zum Bestimmen einer Position des Bereichs von Interesse und einer Position des Messbereichs umfasst.

7. Temperaturüberwachungsvorrichtung nach Anspruch 1, wobei die Temperaturschätzeinheit angepasst ist, um Wärmesenken innerhalb des Gewebes auf der Grundlage der medizinischen Bilder zu erfassen, und wobei die Temperaturschätzeinheit konfiguriert ist, um das Wärmeübertragungsmodell auf der Grundlage der erfassten Wärmesenken anzupassen.

8. Temperaturüberwachungsvorrichtung nach Anspruch 1, wobei die Temperaturschätzeinheit konfiguriert ist, um Parameter des Wärmeübertragungsmodells auf der Grundlage von Ultraschall-Temperaturmessungen in verschiedenen Messbereichen anzupassen.

9. Temperaturüberwachungsvorrichtung nach Anspruch 1, wobei die Temperaturbeaufschlagungseinheit angepasst ist, um die in das Gewebe eingebrachte Heizleistung auf der Grundlage der geschätzten Temperatur im Bereich von Interesse zu steuern.

10. Temperaturüberwachungsvorrichtung nach Anspruch 1, wobei die Ultraschalleinheit angepasst ist, um eine Position des Messbereichs innerhalb des Gewebes auf der Grundlage einer erwarteten Temperatur des Gewebes zu definieren, die auf der Grundlage des Wärmeübertragungsmodells bestimmt wird.

11. Temperaturüberwachungsvorrichtung nach Anspruch 1, wobei die Temperaturbeaufschlagungseinheit angepasst ist, um einen Heizleistungsimpuls in das Gewebe einzubringen, und wobei die Temperaturschätzeinheit angepasst ist, um Parameter des Wärmeübertragungsmodells auf der Grundlage einer von der Ultraschalleinheit bestimmten Temperatur in dem durch den Heizleistungsimpuls erzeugten Messbereich zu bestimmen.

12. Temperaturüberwachungsvorrichtung nach Anspruch 1, wobei die Temperaturbeaufschlagungseinheit eine Sonde (14) zum Einbringen der Wärmeleistung in das Gewebe umfasst, wobei die Sonde eine Temperaturmessvorrichtung (30) zum Messen der Temperatur des Gewebes umfasst.

13. Temperaturüberwachungsvorrichtung nach Anspruch 12, wobei die Temperaturanwendungseinheit angepasst ist, um einen Heizleistungsimpuls in das Gewebe einzubringen, und wobei die Ultraschalleinheit angepasst ist, um die Ultraschalltemperaturmessung auf der Grundlage der von der Temperaturmessvorrichtung der Sonde gemessenen Temperatur zu kalibrieren.

14. Temperaturüberwachungsvorrichtung nach Anspruch 1, wobei die medizinischen Bilder Ultraschallbilder, Computertomographiebilder und/oder Magnetresonanztomographiebilder sind, die von einer Bildeinheit (50) bereitgestellt werden.

## Revendications

1. Appareil de surveillance de température (40) pour surveiller une température à l'intérieur d'un tissu (10) au cours d'un procédé d'ablation thermique, comprenant :
- une unité d'application de température (42) configurée pour introduire de l'énergie de chauffage dans le tissu afin de chauffer le tissu,
- une unité ultrasonique (44) configurée pour émettre et recevoir des ondes ultrasoniques et configurée pour déterminer une température dans une région de mesure (22, 24) du tissu sur la base d'une détection d'onde de cisaillement ultrasonique au cours du procédé d'ablation, et
- une unité d'estimation de température (46) comprenant un modèle de transfert thermique (48) configuré pour estimer une température dans une région d'intérêt (26) à l'intérieur du tissu sur la base de la température déterminée et du modèle de transfert thermique, dans lequel le modèle de transfert thermique est basé sur des images médicales du tissu.

2. Appareil de surveillance de température selon la revendication 1, dans lequel l'unité ultrasonique est à même de définir la région de mesure à l'intérieur du tissu sur la base des images médicales.

3. Appareil de surveillance de température selon la revendication 1, dans lequel l'unité d'application de température est à même de déterminer l'énergie de chauffage introduite dans le tissu sur la base des images médicales.

4. Appareil de surveillance de température selon la revendication 1, dans lequel la région de mesure comprend au moins un plan de mesure.

5. Appareil de surveillance de température selon la revendication 1, dans lequel la région de mesure est formée séparément de la région d'intérêt.

6. Appareil de surveillance de température selon la revendication 1, dans lequel l'unité d'estimation de température comprend un capteur de position (28) pour déterminer une position de la région d'intérêt et une position de la région de mesure.

7. Appareil de surveillance de température selon la revendication 1, dans lequel l'unité d'estimation de température est à même de détecter des puits de chaleur à l'intérieur du tissu sur la base des images médicales et dans lequel l'unité d'estimation de température est configurée pour s'adapter au modèle de transfert thermique sur la base des puits de chaleur détectés.

8. Appareil de surveillance de température selon la revendication 1, dans lequel l'unité d'estimation de température est configurée pour s'adapter à des paramètres du modèle de transfert de chaleur sur la base de mesures de température ultrasoniques dans différentes régions de mesure.

9. Appareil de surveillance de température selon la revendication 1, dans lequel l'unité d'application de température est à même de commander l'énergie de chauffage introduite dans le tissu sur la base de la température estimée dans la région d'intérêt.

10. Appareil de surveillance de température selon la revendication 1, dans lequel l'unité ultrasonique est à même de définir une position de la région de mesure à l'intérieur du tissu sur la base d'une température attendue du tissu déterminée sur la base du modèle de transfert thermique.

11. Appareil de surveillance de température selon la revendication 1, dans lequel l'unité d'application de température est à même d'introduire une impulsion d'énergie de chauffage dans le tissu et dans lequel l'unité d'estimation de température est à même de déterminer des paramètres du modèle de transfert thermique sur la base d'une température déterminée par l'unité ultrasonique dans la région de mesure générée par l'impulsion d'énergie de chauffage.

12. Appareil de surveillance de température selon la revendication 1, dans lequel l'unité d'application de température comprend une sonde (14) pour introduire l'énergie de chauffage dans le tissu, dans lequel la sonde comprend un dispositif de mesure de température (30) pour mesurer la température du tissu.

13. Appareil de surveillance de température selon la revendication 12, dans lequel l'unité d'application de température est à même d'introduire une impulsion d'énergie de chauffage dans le tissu et dans lequel l'unité ultrasonique est à même d'étalonner la mesure de température ultrasonique sur la base de la température mesurée par le dispositif de mesure de température de la sonde.

14. Appareil de surveillance de température selon la revendication 1, dans lequel des images médicales sont des images ultrasoniques, des images tomodensitométriques et/ou des images tomographiques à résonance magnétique fournies par une unité d'images (50).
